(19) Europäisches Patentamt
European Patent Office
Office européen des brevets

(11) Numéro de publication: **0 511 126 B1**

# (12) FASCICULE DE BREVET EUROPEEN

(45) Date de publication de fascicule du brevet: **28.12.94**

(51) Int. Cl.$^{5}$: **C07C 55/14**, C07C 51/14, C07C 69/44, C07C 67/38

(21) Numéro de dépôt: **92420118.9**

(22) Date de dépôt: **13.04.92**

## (54) Procédé de préparation de l'acide adipique par hydrocarboxylation d'acides penténiques.

(30) Priorité: **26.04.91 FR 9105542**

(43) Date de publication de la demande:
**28.10.92 Bulletin 92/44**

(45) Mention de la délivrance du brevet:
**28.12.94 Bulletin 94/52**

(84) Etats contractants désignés:
**BE DE ES FR GB IT NL**

(56) Documents cités:
**EP-A- 0 493 273**
**US-A- 3 816 489**

**CATALYSIS REVIEWS, vol. 23, nos. 1-2, 1981, pages 89-105, New York, US; D. FORSTER et al.: "Mechanistic pathways in the catalysis of olefin hydrocarboxylation by rhodium, iridium, and cobalt complexes"**

(73) Titulaire: **RHONE-POULENC CHIMIE**
**25, Ouai Paul Doumer**
**F-92408 Courbevoie Cédex (FR)**

(72) Inventeur: **Grosselin, Jean-Michel**
**12, allée des Erables**
**F-69340 Francheville (FR)**
Inventeur: **Denis, Philippe**
**16, allée des Troenes**
**F-69150 Decines (FR)**
Inventeur: **Metz, François**
**6, rue de la Salle des Fêtes**
**F-69390 Vernaison (FR)**

(74) Mandataire: **Vignally, Noel et al**
**RHONE-POULENC CHIMIE,**
**Direction de la Propriété Industrielle,**
**Centre de Recherches des Carrières,**
**B.P. 62**
**F-69192 Saint-Fons Cédex (FR)**

**EP 0 511 126 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne un procédé de préparation de l'acide adipique par hydrocarboxylation d'acides penténiques, c'est-à-dire par réaction de l'eau et de l'oxyde de carbone sur au moins un acide penténique.

Dans le brevet américain 3,579,551 il a été proposé un procédé de préparation d'acides carboxyliques par réaction de composés à insaturation éthylénique avec de l'oxyde de carbone et de l'eau, en présence d'une composition catalytique comprenant essentiellement des composés ou complexes de l'iridium, et un promoteur iodé. Les composés à insaturation éthylénique sont convertis sélectivement en acides carboxyliques (linéaires et branchés) par la réaction en cause conduite, de préférence en phase liquide, à une température comprise entre 50 et 300 ° C (de préférence entre 125 et 225 ° C), sous des pressions partielles d'oxyde de carbone avantageusement comprises entre 5 et 3000 psia voire, entre 25 et 1000 psia.

N'importe quelle source d'iridium paraît pouvoir être utilisée et diverses sources de promoteurs iodés sont mentionnées ; le rapport atomique I/Ir peut varier dans de larges limites (1:1 à 2500:1) et de préférence entre 3:1 et 300:1.

Le milieu réactionnel liquide peut renfermer n'importe quel solvant compatible avec le système catalytique, les acides monocarboxyliques en $C_2$-$C_{20}$ étant les solvants préférés.

L'exemple 1 réalisé au départ de propylène montre qu'un tel système favorise la formation d'acides carboxyliques branchés (isobutyrique).

L'exemple 19 réalisé au départ d'héxène-1 confirme l'importance de la proportion d'acides carboxyliques branchés ainsi obtenus.

Cet inconvénient (manque de sélectivité en acide carboxylique linéaire) n'a pas échappé au titulaire du brevet américain précité. En effet, dans le brevet américain n° 3,816,489, il est proposé de mettre en oeuvre la réaction en cause avec un rapport atomique I/Ir compris entre 3:1 et 100:1 pour obtenir des acides carboxyliques terminaux de manière prépondérante.

Si l'intérêt de principe de telles techniques n'est pas remis en cause, dans le cas de matières premières choisies parmi les composés à insaturation oléfinique non fonctionnalisés et en particulier dans le cas des oléfines elles-mêmes, la transposition de ces techniques à des matières premières comportant en plus de l'insaturation éthylénique, une fonction réactive dans les conditions de la réaction en cause, se heurte à de nombreuses difficultés.

En particulier, les premières tentatives de transpositions réalisées par la Demanderesse au départ d'acides penténiques se sont soldées au moins par un échec partiel, des réactions différentes de celle spécifiquement visée se produisant au détriment voire en remplacement total de celle-ci en raison de la présence d'une fonction -COOH sur la matière première à insaturation éthylénique.

Dans le brevet EP-A-0493273 relatif à un procédé d'hydroxycarbonylation d'acides penténoïques en acide adipique, en présence de rhodium ou d'un composé du rhodium, d'un promoteur iodé et d'un co-catalyseur choisi dans le groupe constitué par l'iridium, le ruthénium, l'osmium et leurs composés, un essai témoin (b) a été réalisé avec l'acide pentène-3 oïque, en utilisant le trichlorure d'iridium comme catalyseur, l'iodure d'hydrogène comme promoteur et l'acide acétique comme solvant, le rapport atomique I/Ir étant de 5,7. Cet essai a produit l'acide adipique avec un rendement très faible de 1,2 %.

Dans un tel contexte il serait manifestement souhaitable de pouvoir disposer d'un procédé offrant simultanément de bonnes performances dans la réaction de carbonylation visée et une sélectivité appréciable en acide adipique.

La présente invention a donc pour objet un procédé de préparation de l'acide adipique par réaction de l'eau et de l'oxyde de carbone sur au moins un acide penténique, en présence d'un catalyseur à base d'iridium et d'au moins un promoteur iodé, à une température élevée, sous une pression supérieure à la pression atmosphérique, dans au moins un acide carboxylique choisi parmi les acides carboxyliques aliphatiques saturés, les acides carboxyliques aliphatiques éthylèniques et les acides carboxyliques aromatiques, comportant au maximum 20 atomes de carbone, le rapport atomique I/Ir étant inférieur à 10, et lorsque le catalyseur est le trichlorure d'iridium et le solvant l'acide acétique, le rapport atomique I/Ir étant alors en outre différent de 5,7.

Par acide penténique, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié compte-tenu de son accessibilité et des résultats satisfaisants auxquels il conduit dans le cadre du présent procédé.

Le procédé selon la présente invention nécessite la présence d'un catalyseur à base d'iridium. Diverses sources d'iridium sont susceptibles d'être mises en oeuvre.

A titre d'exemple de sources d'iridium susceptibles de convenir à la mise en oeuvre du présent procédé on peut citer :

Ir métallique ; $Ir\ O_2$ ; $Ir_2\ O_3$ ;
$Ir\ Cl_3$ ; $Ir\ Cl_3$ , $3\ H_2O$ ;
$Ir\ Br_3$ ; $Ir\ Br_3$ , $3H_2O$ ;
$Ir\ I_3$ ;
$Ir_2(CO)_4Cl_2$ ; $Ir_2(CO)_4I_2$ ;
$Ir_2(CO)_8$ ; $Ir_4(CO)_{12}$ ;
$Ir_2(CO)[P(C_6H_5)_3]_2\ I$ ;
$Ir(CO)[P(C_6H_5)_3]_2\ Cl$ ;
$Ir[P(C_6H_5)_3]_3I$ ;
$HIr[P(C_6H_5)_3]_3(CO)$ ;
$Ir(acac)(CO)_2$
$[IrCl(Cod)]_2$
(Cod : cyclooctadiène-1,5 ; acac : acétylacétonate)

Conviennent plus particulièrement à la mise en oeuvre du présent procédé : $[IrCl(Cod)]_2$ ; $Ir_4(CO)_{12}$ et $Ir(acac)(CO)_2$.

La quantité d'iridium à mettre en oeuvre peut varier dans de larges limites.

En général, une quantité exprimée en mole d'iridium métallique par litre de milieu réactionnel comprise entre $10^{-4}$ et $10^{-1}$ conduit à des résultats satisfaisants. Des quantités inférieures peuvent être mise en oeuvre ; on observe toutefois que la vitesse de réaction est faible. Des quantités supérieures n'ont d'inconvénients qu'au plan économique.

De préférence, la concentration d'iridium est comprise entre $5.10^{-4}$ et $10^{-2}$ (inclus) mol/l.

Par promoteur iodé on entend dans le cadre du présent procédé HI et les composés organoiodés capables de générer HI dans les conditions réactionnelles et, en particulier les iodures d'alkyles en $C_1$-$C_{10}$, l'iodure de méthyle étant plus particulièrement préconisé.

Selon une caractéristique essentielle du présent procédé, la quantité de promoteur iodé à mettre en oeuvre est telle que le rapport molaire I/Ir soit inférieur à 10. Il s'avère souhaitable que ce rapport soit supérieur ou égal à 0,1. De préférence, le rapport molaire I/Ir est compris entre 1 et 5 (inclus).

La présence d'eau est indispensable à la conduite du procédé selon la présente invention. En général, la quantité d'eau à mettre en oeuvre est telle que le rapport molaire eau/acide(s) penténique(s) soit compris entre 0,01 et 10 (inclus).

Une quantité supérieure n'est pas souhaitable en raison de la perte d'activité catalytique observée. Le rapport molaire eau/acide(s) penténique(s) dans le milieu réactionnel peut être instantanément inférieur à la valeur minimale indiquée précédemment, si l'on procède par exemple à l'injection continue de l'eau, plutôt qu'à son introduction avec les autres charges avant la réaction d'hydrocarboxylation.

De préférence, le rapport molaire eau/acide(s) penténique(s) est compris entre 0,01 et 1 (inclus), la remarque précédente concernant la valeur minimale étant également valable.

Selon une autre des caractéristiques essentielles de la présente invention, la réaction est mise en oeuvre dans au moins un acide carboxylique choisi parmi les acides carboxyliques aliphatiques saturés, les acides carboxyliques aliphatiques éthylèniques et les acides carboxyliques aromatiques, comportant au maximum 20 atomes de carbone.

La nature précise de l'acide carboxylique n'est pas critique dans le cadre du présent procédé, pour autant que cet acide soit à l'état liquide dans les conditions réactionnelles.

A titre d'exemples d'acides carboxyliques susceptibles de convenir à la mise en oeuvre du procédé selon l'invention, on peut citer :

l'acide acétique, l'acide propionique, l'acide butyrique, l'acide valérique, l'acide adipique, l'acide pentène-2-oïque, l'acide pentène-3-oïque, l'acide pentène-4-oïque, les mélanges de 2 ou 3 des acides penténiques précédents, l'acide benzoïque et l'acide phénylacétique.

De préférence on recourt à un acide carboxylique aliphatique saturé en $C_2$-$C_6$. L'acide acétique convient plus particulièrement à la mise en oeuvre de la présente invention. L'acide adipique, qui est le composé dont la préparation est visée par le procédé de l'invention, convient également bien.

Une autre variante préférentielle de l'invention consiste à utiliser comme acides carboxyliques, l'acide pentène-2-oïque, l'acide pentène-3-oïque, l'acide pentène-4-oïque et les différents mélanges de ces acides.

La quantité d'acide carboxylique présente dans le milieu réactionnel peut varier dans de larges limites, par exemple de 10 à 99 % (inclus) en volume du milieu réactionnel. De préférence cette quantité est comprise entre 30 et 99 % en volume (inclus). Lorsque l'acide carboxylique utilisé est choisi parmi les acides penténiques, on se situe évidemment dans la zone supérieure des valeurs indiquées précédemment.

Comme indiqué en tête du présent mémoire, la réaction est conduite sous une pression supérieure à la pression atmosphérique et en présence d'oxyde de carbone.

On peut utiliser de l'oxyde de carbone sensiblement pur ou de qualité technique, tel qu'il se présente dans le commerce.

La réaction est conduite de préférence en phase liquide, la pression totale pouvant varier dans de larges limites et la température de réaction étant généralement comprise entre 100 et 240 ° C et, de préférence entre 160 et 190 ° C.

La pression partielle de l'oxyde de carbone est généralement comprise entre 2 et 250 bar et pour une bonne mise en oeuvre du procédé selon l'invention, elle sera comprise entre 5 et 100 bar.

Le milieu réactionnel renferme l'acide carboxylique aliphatique saturé, l'acide carboxylique aliphatique éthylènique ou l'acide carboxylique aromatique, de l'eau, une ou des sources d'iridium, un ou des promoteurs iodés et, le cas échéant tout ou partie du ou des acides penténiques engagés et des produits de la réaction.

En fin de réaction ou du temps imparti à celle-ci, on sépare l'acide adipique par tout moyen approprié par exemple par cristallisation et/ou distillation de l'acide carboxylique.

Les exemples ci-après illustrent la présente invention.

EXEMPLE 1 :

Dans une ampoule de verre préalablement purgée à l'argon, on introduit :

33,6 mg (0,1 mmol) d'iridium sous forme de $[IrCl(Cod)]_2$

0,045 g (0,2 mmol) de HI sous forme d'une solution aqueuse à 57 % (en poids)

0,5 g ( 28 mmol) d'eau

2,0 g ( 20 mmol) d'acide pentène-3-oïque

10 $cm^3$ d'acide acétique.

L'ampoule est placée dans un autoclave de 125 ml.

L'autoclave est fermé hermétiquement, placé dans un four agité. et raccordé à l'alimentation de gaz sous pression. On admet à froid 2 bar de CO et on chauffe à 175 ° C en 20 minutes. Lorsque cette température est atteinte, on régule la pression à 20 bar.

Après une durée de réaction de 20 minutes, l'agitation et le chauffage sont arrêtés ; l'autoclave est alors refroidi et dégazé.

La solution réactionnelle est analysée par chromatographie en phase gazeuse et par chromatographie liquide haute performance.

Les quantités de produits formés (rendement molaire par rapport à l'acide pentène-3 oïque transformé) sont les suivantes :

acide valérique (Pa) : 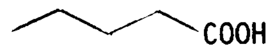 = 3,5 %

acide pentène-4 oïque (P4) : 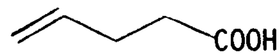 = 1 %

acide pentène-3 oïque (P3) : 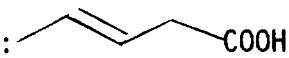 = 30 %

acide pentène-2 oïque (P2) : 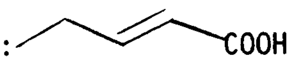 = 6,5 %

γ-valérolactone : 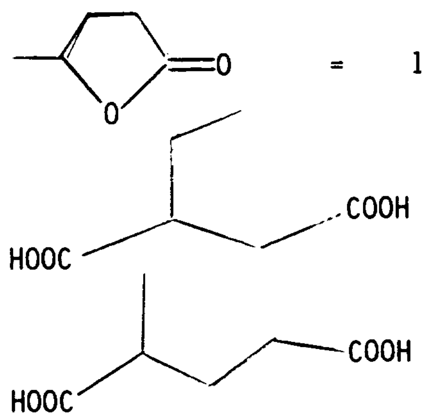 = 10 %

acide éthylsuccinique (A3) : = 3,5 %

acide méthylglutarique (A2) : = 16,5 %

acide adipique (A1) : 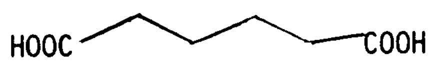 = 59 %

Le taux de linéarité (L) est de 75 %
Le taux de transformation de l'acide pentène-3 oïque (TT) est de 70 %.

EXEMPLES 2 et 3 :

Selon le mode opératoire décrit pour l'exemple 1, on réalise une première série d'essais, en ne modifiant que la quantité d'HI chargée.

Les conditions particulières ainsi que les résultats obtenus toutes conditions égales par ailleurs sont rassemblés dans le tableau (I) ci-après, dans lequel les conventions utilisées sont les mêmes que pour l'exemple 1 :

TABLEAU I

| Réf. | HI/Ir | TT % | AI % | L % | M4L % |
|---|---|---|---|---|---|
| 2 | 1 | 36 | 49 | 65 | 10 |
| 1 | 2 | 70 | 59 | 75 | 10 |
| 3 | 3 | 50 | 48 | 70 | 20 |

EXEMPLE 4

Selon le mode opératoire décrit pour l'exemple 1, on répète l'exemple 3 en diminuant la quantité d'iridium sous forme de $[IrCl(Cod)]_2$ : 12,6 mg (0,0375 mmol), les quantités des différentes autres charges demeurant les mêmes.

Le rapport molaire HI/Ir est ainsi de 8.

La durée de réaction en température est de 60 minutes.

Les résultats obtenus sont indiqués ci-après (avec les conventions utilisées pour l'exemple 1) :

| | |
|---|---|
| - TT de l'acide pentène-3-oïque | 58 % |
| - Al | 5 % |
| - L | 62 % |
| - M4L | 92 % |

EXEMPLES 5 à 12 :

Dans l'autoclave et selon le mode opératoire decrits ci-avant on réalise une série d'essais sur une charge renfermant :

- 0,1 mmol d'iridium sous forme d'$Ir_4(CO)_{12}$
- 0,2 mmol d'HI sous forme d'une solution aqueuse à 57 % poids)
- 20 mmol d'acide pentène-3 oïque
- une quantité variable d'eau
- 10 cm3 d'acide acétique.

Les conditions particulières ainsi que les résultats obtenus en 20 minutes de réaction à la température de 175 °C sont rassemblés dans le tableau II ci-après dans lequel P(CO) représente la pression partielle de l'oxyde de carbone en température.

TABLEAU II

| Réf. | H20 mmol | P(CO) bar | TT % | A1 % | L % | M4L % |
|---|---|---|---|---|---|---|
| 5 | 17 | 20 | 60 | 62,5 | 78 | 10 |
| 6 | 29 | " | 70 | 65,5 | 81 | 8,5 |
| 7 | 67 | " | 37 | 41 | 72 | 24 |
| 8 | 193 | " | 34 | 40 | 67 | 20 |
| 9 | 29 | 5 | 51 | 21 | 72 | 6,5 |
| 10 | " | 10 | 82 | 63 | 84 | 10 |
| 11 | " | 15 | 68 | 63 | 81 | 10,5 |
| 12 | " | 50 | 57 | 59 | 78 | 10 |

EXEMPLE 13 :

On reproduit l'exemple 6 ci-avant en ne modifiant que la température (190 °C).
Les résultats obtenus toutes conditions égales par ailleurs sont les suivants :
TT (%) = 84
A1 (%) = 39
L (%) = 76
M4L(%) = 9

EXEMPLE 14 :

On reproduit l'exemple 6 ci-avant en ne modifiant que la quantité-d'iridium chargée (0,05 mmol).
Les résultats obtenus toutes conditions égales par ailleurs sont les suivants :
TT (%) = 26
A1 (%) = 39
L (%) = 63
M4L(%) = 23

EXEMPLE 15 :

On reproduit l'exemple 6 ci-avant en doublant la quantité d'iridium chargée.
Les résultats obtenus toutes conditions égales par ailleurs sont les suivants :
TT (%) = 73
A1 (%) = 56
L (%) = 82
M4L(%) = 9

ESSAI TEMOIN :

On reproduit l'exemple 6 ci-avant en remplaçant HI par une quantité équivalente d'HBr.
Les résultats obtenus toutes conditions égales par ailleurs sont les suivants :
TT (%) = 9
A1 (%) = 0
M4L(%) = 83

EXEMPLES 16 et 17 :

On reproduit l'exemple 6 ci-avant en ne changeant que la nature de l'acide penténique chargé.

Les conditions particulières ainsi que les résultats obtenus toutes conditions égales par ailleurs sont rassemblés dans le tableau III ci-après :

TABLEAU III

| Réf. | Acide chargé | TT % | A1 % | L % | M4L % | PA |
|---|---|---|---|---|---|---|
| 6 | pentène-3 oïque | 70 | 65,5 | 81 | 8,5 | 3,5 |
| 16 | pentène-4 oïque | 100 | 63 | 88 | 14,5 | 3 |
| 17 | pentène-2 oïque | 45 | 54,5 | 88 | 6 | 24 |

EXEMPLE 18 :

Dans une ampoule de verre préalablement purgée à l'argon, on introduit :
33,6 mg (0,1 mmol) d'iridium sous forme de $[IrCl(Cod)]_2$
0,045 g (0,2 mmol) de HI sous forme d'une solution aqueuse à 57 % (en poids)
0,5 g (28 mmol) d'eau
11,9 g (119 mmol) d'acide pentène-3-oïque.
L'ampoule est placée dans un autoclave de 125 ml.

L'autoclave est fermé hermétiquement, placé dans un four agité et raccordé à l'alimentation de gaz sous pression. On admet à froid 2 bar de CO et on chauffe à 175 °C en 20 minutes. Lorsque cette température est atteinte, on régule la pression à 20 bar.

Après une durée de réaction de 55 minutes, l'agitation et le chauffage sont arrêtés et la solution réactionnelle est analysée comme dans l'exemple 1.

On obtient les résultats suivants (avec les conventions utilisées pour l'exemple 1) :

| | |
|---|---|
| - TT de l'acide pentène-3-oïque | 34 % |
| - A1 | 71 % |
| - L | 86 % |
| - M4L | 11 % |

EXEMPLE 19 :

On répète l'exemple 18 avec les charges suivantes :
33,6 mg (0,1 mmol) d'iridium sous forme de $[IrCl(Cod)]_2$

0,045 g (0,2 mmol) de HI sous forme d'une solution aqueuse à 57 % (en poids)
0,5 g ( 28 mmol) d'eau
2,0 g ( 2 mmol) d'acide pentène-3-oïque
10 g d'acide adipique.
On obtient les résultats suivants (avec les conventions utilisées pour l'exemple 1) :

| | |
|---|---|
| - TT de l'acide pentène-3-oïque | 95 % |
| - A1 (acide adipique formé, à l'exclusion de l'acide chargé au départ) | 77 % |
| - L | 86 % |
| - M4L | 12 % |

EXEMPLE 20 :

On répète l'exemple 18 avec les charges suivantes :
33,6 mg (0,1 mmol) d'iridium sous forme de $[IrCl(Cod)]_2$
0,045 g (0,2 mmol) de HI sous forme d'une solution aqueuse à 57 % (en poids)
0,5 g ( 28 mmol) d'eau
2,0 g ( 2 mmol) d'acide pentène-3-oïque
10 g d'acide pentène-2-oïque.
On obtient les résultats suivants (avec les conventions utilisées pour l'exemple 1) :

| | |
|---|---|
| - TT de l'acide pentène-3-oïque | 40 % |
| - A1 | 59 % |
| - L | 83 % |
| - M4L | 3 % |

## Revendications

1. Procédé de préparation de l'acide adipique par réaction de l'eau et de l'oxyde de carbone sur au moins un acide penténique, en présence d'un catalyseur à base d'iridium et d'au moins un promoteur iodé, à une température élevée, sous une pression supérieure à la pression atmosphérique, dans au moins un acide carboxylique choisi parmi les acides carboxyliques aliphatiques saturés, les acides carboxyliques aliphatiques éthyléniques et les acides carboxyliques aromatiques, comportant au maximum 20 atomes de carbone, le rapport atomique I/Ir étant inférieur à 10, et lorsque le catalyseur est le trichlorure d'iridium et le solvant l'acide acétique, le rapport atomique I/Ir étant alors en outre différent de 5,7.

2. Procédé selon la revendication 1, caractérisé en ce que l'acide penténique est choisi parmi l'acide pentène-3-oïque, l'acide pentène-2-oïque, l'acide pentène-4-oïque et leurs mélanges.

3. Procédé selon l'une des revendications 1 ou 2, caractérisé en ce que le rapport molaire eau/acide(s) penténique(s) est inférieur ou égal à 10.

4. Procédé selon l'une des revendications 1 à 3, caractérisé en ce que l'acide carboxylique est choisi parmi les acides aliphatiques saturés en $C_2$-$C_6$.

5. Procédé selon la revendication 4, caractérisé en ce que l'acide carboxylique est l'acide acétique ou l'acide adipique.

6. Procédé selon l'une des revendications 1 à 3, caractérisé en ce que l'acide carboxylique est choisi parmi l'acide pentène-3-oïque, l'acide pentène-2-oïque, l'acide pentène-4-oïque et les mélanges de deux ou trois d'entre eux.

7. Procédé selon l'une des revendications 1 à 6, caractérisé en ce que l'acide carboxylique représente au minimum 10 % du volume du milieu réactionnel.

8. Procédé selon l'une des revendication 4 à 6, caractérisé en ce que l'acide carboxylique représente de 10 à 99 % (inclus) et de préférence de 30 à 99 % (inclus) du volume du milieu réactionnel.

9. Procédé selon l'une des revendications 1 à 8, caractérisé en ce que la concentration en iridium dans le milieu réactionnel est comprise entre $10^{-4}$ et $10^{-1}$ mol/l (inclus).

10. Procédé selon l'une des revendications 1 à 9, caractérisé en ce que la température de réaction est comprise entre 100 et 240 ° C et de préférence entre 160 et 190 ° C.

11. Procédé selon l'une des revendications 1 à 10, caractérisé en ce que la pression partielle de l'oxyde de carbone est comprise entre 2 et 250 bar et de préférence entre 5 et 100 bar.

12. Procédé selon l'une des revendications 1 à 11, caractérisé en ce que le rapport atomique I/Ir est supérieur ou égal à 0,1.

13. Procédé selon l'une des revendications 1 à 12, caractérisé en ce que le rapport atomique I/Ir est compris entre 1 et 5 (inclus).

**Claims**

1. Process for the preparation of adipic acid by reacting water and carbon monoxide with at least one pentenic acid, in the presence of a catalyst based on iridium and at least one iodine-containing promoter, at a high temperature and at a pressure greater than atmospheric pressure, in at least one carboxylic acid chosen from saturated aliphatic carboxylic acids, ethylenic aliphatic carboxylic acids and aromatic carboxylic acids, containing a maximum of 20 carbon atoms, the I/Ir atomic ratio being less than 10, and when the catalyst is iridium trichloride and the solvent is acetic acid, the I/Ir atomic ratio then additionally being different from 5.7.

2. Process according to claim 1, characterized in that the pentenic acid is chosen from 3-pentenoic acid, 2-pentenoic acid, 4-pentenoic acid and their mixtures.

3. Process according to either of claims 1 and 2, characterized in that the water/pentenic acid(s) molar ratio is less than or equal to 10.

4. Process according to one of claims 1 to 3, characterized in that the carboxylic acid is chosen from saturated $C_2$-$C_6$ aliphatic acids.

5. Process according to claim 4, characterized in that the carboxylic acid is acetic acid or adipic acid.

6. Process according to one of claims 1 to 3, characterized in that the carboxylic acid is chosen from 3-pentenoic acid, 2-pentenoic acid, 4-pentenoic acid and the mixtures of two or three of these.

7. Process according to one of claims 1 to 6, characterized in that the carboxylic acid represents a minimum of 10% of the volume of the reaction medium.

8. Process according to one of claims 4 to 6, characterized in that the carboxylic acid represents from 10 to 99% (inclusive) and preferably from 30 to 99% (inclusive) of the volume of the reaction medium.

9. Process according to one of claims 1 to 8, characterized in that the iridium concentration in the reaction medium is between $10^{-4}$ and $10^{-1}$ mol/l (inclusive).

10. Process according to one of claims 1 to 9, characterized in that the reaction temperature is between 100 and 240 ° C and preferably between 160 and 190 ° C.

11. Process according to one of claims 1 to 10, characterized in that the partial pressure of carbon monoxide is between 2 and 250 bar and preferably between 5 and 100 bar.

12. Process according to one of claims 1 to 11, characterized in that the I/Ir atomic ratio is greater than or equal to 0.1.

13. Process according to one of claims 1 to 12, characterized in that the I/Ir atomic ratio is between 1 and 5 (inclusive).

**Patentansprüche**

1. Verfahren zur Herstellung von Adipinsäure durch Reaktion von Wasser und Kohlenmonoxid mit wenigstens einer Pentensäure in Gegenwart eines auf Iridium basierenden Katalysators und wenigstens eines iodierten Promotors bei erhöhter Temperatur bei einem über dem Atmosphärendruck liegenden Druck in wenigstens einer Carbonsäure, ausgewählt aus den aliphatischen gesättigten Carbonsäuren, den aliphatischen ethylenischen Carbonsäuren und den aromatischen Carbonsäuren mit höchstens 20 Kohlenstoffatomen, wobei das atomare Verhältnis I/Ir unterhalb von 10 liegt, und, wenn der Katalysator Iridiumtrichlorid und das Lösungsmittel Essigsäure ist, das atomare Verhältnis I/Ir dann außerdem verschieden von 5,7 ist.

2. Verfahren nach Anspruch 1, dadurch gekennzeichnet, daß die Pentensäure aus der 3-Pentensäure, der 2-Pentensäure, der 4-Pentensäure und ihren Mischungen ausgewählt ist.

3. Verfahren nach einem der Ansprüche 1 oder 2, dadurch gekennzeichnet, daß das molare Verhältnis Wasser /Pentensäure(n) Kleiner oder gleich 10 ist.

4. Verfahren nach einem der Ansprüche 1 bis 3, dadurch gekennzeichnet, daß die Carbonsäure aus den gesättigten aliphatischen Säuren mit $C_2$-$C_6$ ist.

5. Verfahren nach Anspruch 4, dadurch gekennzeichnet, daß die Carbonsäure Essigsäure oder Adipinsäure ist.

6. Verfahren nach einem der Ansprüche 1 bis 3, dadurch gekennzeichnet, daß die Carbonsäure ausgewählt ist aus der 3-Pentensäure, der 2-Pentensäure, der 4-Pentensäure und den Mischungen aus zwei oder drei von ihnen.

7. Verfahren nach einem der Ansprüche 1 bis 6, dadurch gekennzeichnet, daß die Carbonsäure wenigstens 10 Volumenprozent des Reaktionsmediums ausmacht.

8. Verfahren nach einem der Ansprüche 4 bis 6, dadurch gekennzeichnet, daß die Carbonsäure 10 bis 99% (einschließlich), vorzugsweise 30 bis 99% (einschließlich), des Reaktionsmediums ausmacht.

9. Verfahren nach einem der Ansprüche 1 bis 8, dadurch gekennzeichnet, daß die Konzentration an Iridium in dem Reaktionsmedium zwischen $10^{-4}$ und $10^{-1}$ mol/l (einschließlich) liegt.

10. Verfahren nach einem der Ansprüche 1 bis 9, dadurch gekennzeichnet, daß die Reaktionstemperatur zwischen 100 und 240 °C, vorzugsweise zwischen 160 und 190 °C, liegt.

11. Verfahren nach einem der Ansprüche 1 bis 10, dadurch gekennzeichnet, daß der Partialdruck des Kohlenmonoxids zwischen 2 und 250 bar, vorzugsweise zwischen 5 und 100 bar, liegt.

12. Verfahren nach einem der Ansprüche 1 bis 11, dadurch gekennzeichnet, daß das atomare Verhältnis I/Ir größer oder gleich 0,1 ist.

13. Verfahren nach einem der Ansprüche 1 bis 12, dadurch gekennzeichnet, daß das atomare Verhältnis I/Ir zwischen 1 und 5 (einschließlich) liegt.